# DEMANDE DE BREVET EUROPEEN

(11) **EP 0 552 503 A1**
(43) Date de publication de la demande: **28.07.1993**
(21) Numéro de dépôt: 92200054.2
(22) Date de dépôt: 23.01.1992
(51) Int. Cl.: A61K 33/04

(54) **Composition de sulfate de barium colloidal pour le traitement des néoplasmes du poumon**

(71) Demandeur: Kowalski, Romuald, Dr., Matejki (PL)
(72) Inventeur: Kowalski, Romuald, Dr., Matejki (PL)

(57) **Abrégé**

Le médicament Laitvit- Nèoplasme - R.K. grâce à ses propriétés surtout antitumorigénes et accroissant l'épi- phylaxie de l'organisme ainsi que possédant les proprié- tés anti - inflammatoires remarquables a son application dans le traitement des tumeurs malignes surtout des tumeurs:
des poumons,des organes génitaux de femmes,du pancréas et du rectum.

De bons résultats ont obtenus dans le traitement de tumeurs malignes du système lymphatiques,et des tumeurs du système osseux ainsi que des tumeurs malignes de la peau.Ce médicament peut être utilisé durant plusieurs années sans provoquer d'effets secondaires.Il peut être appliqué à chaque âge.Il peut être aussi appliqué chez les femmes encentes.

## Description

Le mèdicament Laitvit - Nèoplasme - R.K. - rend des services excellentes dans le traitement des nèoplasmes malignes.

Compte tenu de ses propriété analgétiques peut très bien remplacer d'autres médicament à une forte action analgétiques tels que p.ex.la morfine sans causer,en même temps,de lésion des organes internes.

En outre,ces médicaments posédent les propriétés particulières anti - inflammatoires qui,très souvant, accompagnent les lésions nèoplasiques,d'ou leur grande utilité dans le liqui- dation de l'état inflammatoires accompagnant ces lésions, ce qui favorise à son tour, un meilleur accès de la substance détruisant ce néoplasme.

L'une des propriètés essentieles de ce médicament s'est l'augmentation de la vascularisation de l'organe ce qui favorise l'augmentation de la résorption des cellules détrui- tes,aussi bien néoplastiquees que d'autres.

Cette élimination rapide des cellules nécrotiques rend plus facile l ' accés du médicament active - agissant / sulfate de baryum/ à des cellules altérées pathologiquement toujours plus neuves.

Le médicament Laitvit - Nèoplasme - R.K.,parmi d'autres propriétés,posséde aussi les propriétés augmentant les forces immunologiques de l'organisme,
- il augmente l'appétit,
- augmente la force de l'organisme,
- reléve le moral donc il incite l'organisme à une coppèration dans la lutte contre ce néoplasme.

Ce médicament rend des services exellentes dans le traitement des conséquences des radiolésions des tissus et,dans plusieurs cas,il est plus efficace que l'énergie rayonnante.

Il donne des effets parfaits dans la prévention des réchutes de la maladie néoplasiques succédant une intervention chirurgi- cale.

Il n'agit que sur les cellules changées pathologiquement, accroit la régéneration des cellules de l'entourage sain.

L'action est la plus efficace quand ce reméde agit directe- ment sur le néoplasme lui - même car les foyers metastatique du sarcome des os dans la peau p.exa se fondent sous l'enflu- ance de son action comme la neige sous le soleil.

Par contre,partout là où cet accès est difficille ou assez difficille,l'action de ce médicament,elle aussi, est plus petite ou minimale.

Il agit très efficacement sur les néoplasmes des types différentes à partir des tumeurs du poumon jusqu'à nèoplasmes des organes genitaux de l'utérus.

Ce médicament peut être bien appliqué comme associé avec la chimie antitumorigéne utilisée.

Les patients tolérent mieux de la chimie quand elle est,en même temps,accompagnée du médicament: Laitvit - Nèoplasme -R.K.

L'application du médicament Laitvit - Nèoplasme - R.K. dans le cas d'affections néoplasiques emporte d'autant plus sur d'autres médicaments utilisés jusqu'à présent que, dans les cas desespérés accompagnée de vives douleurs, ce médicament apporte un soulagement à ces malades et fait que les derniers moments de leur vie ne sont plus des cauchemars de martyre, de souffrance et les malades quittent ce monde en paix et même sans savoir quand.

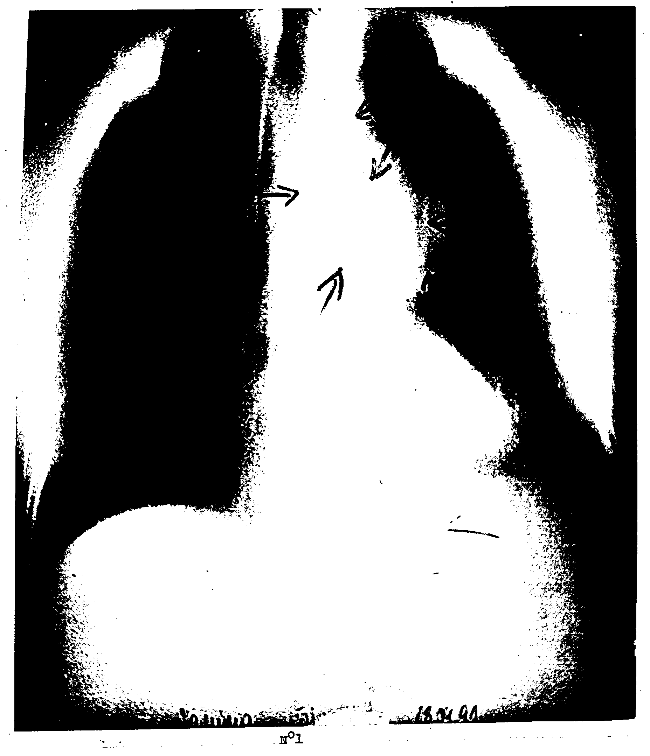

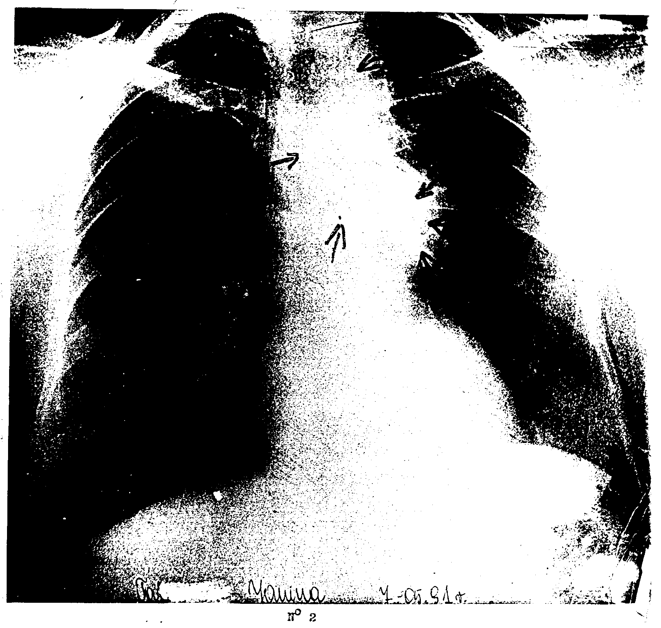

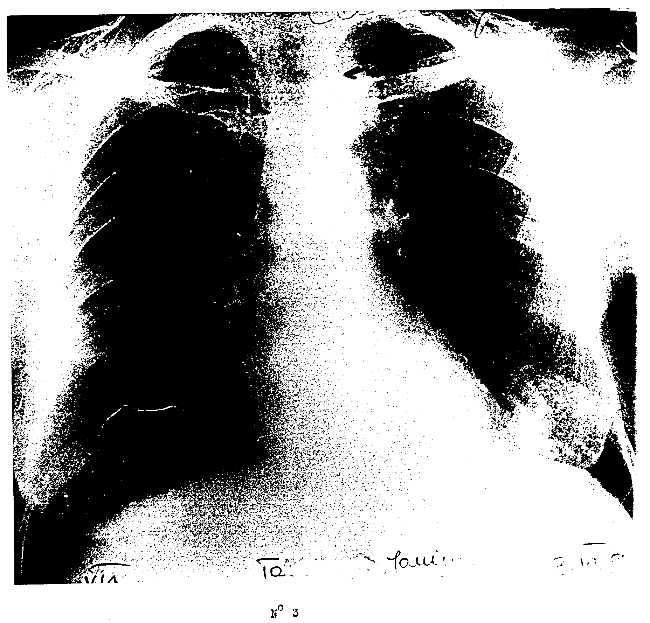

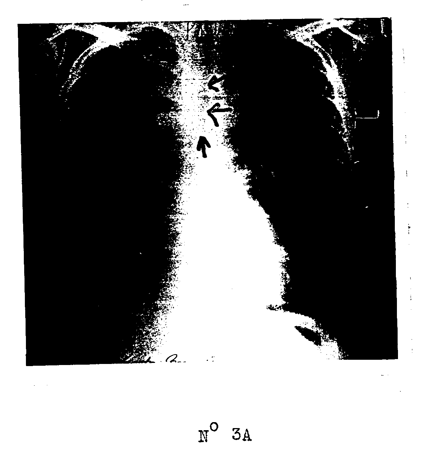

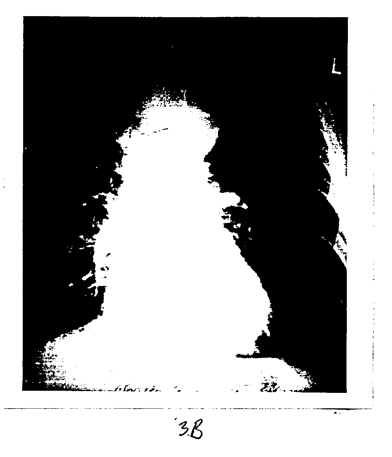

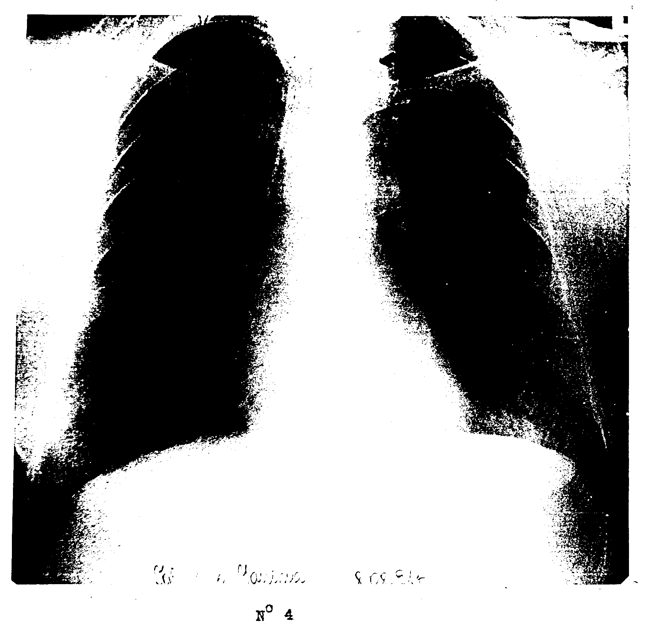

## Revendications

1. Comme la substance active,dans la composition du médica- ment Laitvit - Nèoplasme - R.K. entre la substance active: sulfate de baryum.

2. Pour que la substance active mentionnée dans le point 1 puisse être appliquée sans nuire à la santé de l'orga-nisme soigné elle doit:
a/ subir une dispersion colloidale.
b/ être gardée en pectine pendant une semaine,
c/ séchée dans une chambre termique à température de 60 - 70^{o} C jusqu'à ce qu'elle devienne une substance compacte pareille à un roc blanc.

3. La substance compacte obtenue dans le point 2c ci- dessus est soumise à une nouvelle trituration pour devenir une une poudre dont les grains ont: 0,1 - 0,01 mm d'épaisseur et elle porte le nom de Laitvit -Sulfate - Néoplasme - R.K.

4. le mélange des combinaisons chimiques mentionné dans le point 3 sert à la confection:
1.du médicament nommé Laitvit - Nèoplasme - R.K. en poudre,
2.du médicament Laitvit - Nèoplasme - R.K. en tablettes,
3. du médicament Laitvit - Nèoplasme - R.K. en suppoistoires,
4.du médicament Laitvit - Nèoplasme - R.K. en liquide,
5.du médicament Laitvit - Nèoplasme - R.K. en pommade,
6.du médicament Laitvit - Nèoplasme - R.K. en suspension d'huile.

5. Afin d'augmenter la sécurité et pour éviter des effets secondaires éventuels la substance active sulfate de baryum dans le médicament Laitvit - Néoplasme - R.K. mentionnée dans les points: 1 et 2 est administrée dans la protection de lait de vache en poudre.
